# EUROPEAN PATENT APPLICATION

(11) **EP 1 382 321 A1**
(43) Date of publication of application: **21.01.2004**
(21) Application number: 02028975.7
(22) Date of filing: 24.12.2002
(51) Int. Cl.: A61J 3/00, B01F 13/10

(54) **Method and apparatus for dispensing a customized pharmaceutical mixture**

(30) Priority: 15.07.2002 US 194001
(71) Applicant: The Egg Factory L.L.C., Roanoke, VA 24017 (US)
(72) Inventor: Gentiluomo, Andrew, Roanoke Virginia 24018 (US); Zelikovich, Mikhail, Roanoke Virginia 24018 (US); Banko, Heidi, Herndon Virginia 20171 (US); Elliott, A. Blair, Manchester New Hampshire 03104 (US); Skiles, Jeffrey F., Pilot Virginia 24138 (US)
(74) Representative: VOSSIUS & PARTNER

(57) **Abstract**

A method and apparatus for dispensing a customized pharmaceutical mixture. In an embodiment of a method of the present invention, health information particular to a user of the mixture may be received interactively from the user, amounts of the components of the mixture to be dispensed may be determined based on the health information, and the amounts may be automatically mixed to produce the mixture. In an embodiment of an apparatus of the present invention, the apparatus may include a plurality of containers, that contain multiple pharmaceutical substances, and a controller that selects the containers containing the substances that make up the mixture, adjusts the amounts of the substances to be dispensed, and controls the amounts dispensed to produce the mixture. The apparatus dispenses these amounts based on health information particular to a user of the mixture. Accordingly, the user receives a customized pharmaceutical mixture.

## Description

### RELATED DOCUMENTS

This application claims priority to U.S. provisional application no. 60/304,755, filed July 13, 2001, the entire contents of which are incorporated herein by reference.

Embodiments of the present invention relate to pharmaceutical dispensing devices and, more particularly, to a method and an apparatus for dispensing a customized pharmaceutical mixture for individual users, with potential use in a multi-user environment.

Various devices are known for dispensing different types of pharmaceutical substances. One problem with such devices in general is their inability to dispense substances that are customized in real time for individual users, with potential use in a multi-user environment. In such an environment, each user requires a unique mix of different pharmaceutical substances (e.g., nutritional supplements) based on various unique personal factors (e.g., age, gender, weight, daily level of physical activity, and current health status). Known devices are unable to provide each individual user, regardless of age, gender, or other factors, with a substance blend that is customized for said individual user.

Instead, known dispensing devices are usually concerned with the act of dispensing different final composition pharmaceutical substances in their original form, without the ability for the user to change or otherwise customize the blend of different substances at the time it is dispensed. In this respect, known devices typically fail to take into account the unique personal factors of each user and thus, fail to mix a customized pharmaceutical substance mixture for individual users, with potential of being used in a multi-user environment, based on these factors.

This is a concern in nutritional supplement dispensing, for example. Research shows widespread misuse of nutritional supplements, with users taking too much or too little or the wrong combination of nutritional supplements, thus, increasing the potential for resulting health risks. When taking a generic multi-vitamin, the individual user receives more or less of certain nutritional supplements than what is required by said user. Known dispensing devices do not correct this problem. Instead, known devices arbitrarily dispense the amounts of these and other pharmaceutical substances.

For example, U.S. Pat. No. 5,568,880 to DiBartolomeo discloses a compartmentalized vitamin dispensing system comprising of a plurality of dispensing tubes, and a plurality of dispensing knobs, each including a valve coupled below the dispensing tubes, each valve including a device to retain and dispense materials residing in the dispensing tubes upon turning of the knobs. And, U.S. Pat. No. 4,775,077 to Capotorto discloses a vitamin dispenser comprising of a plurality of slots for holding different types of vitamins, viewing windows for providing an indication as to when a particular type of vitamin should be refilled, and manual release levers for dispensing different types of vitamins when desired. These types of known devices, as in DiBartolomeo and Capotorto, cannot be customized for individual users, with potential use in a multi-user environment. In addition, these devices dispense whole tablets and have no mechanism for controlling the amount of a substance dispensed based on the unique factors (e.g., age, gender, weight, daily level of physical activity, and current health status) of each user.

U.S. Pat. No. 4,207,992 to Brown discloses a timed medicine dispenser comprising of a dispensing wheel having a plurality of medication storage compartments, and a timing mechanism for dispensing medicine on a timed basis for a patient to remind the patient to take the medication. U.S. Pat. No. 4,223,801 to Carlson discloses an automatic periodic drug dispensing system comprising of a clock mechanism, and a signal responsive paging device all of which are arranged to indicate when and which pharmaceutical preparation is to be taken. And, U.S. Pat. No. 3,968,900 to Stambuk discloses a timed medicant dispensing device comprising of an electrically operated device capable of holding first and second hollow plastic balls that contain predetermined numbers of tablets or capsules of first and second medicants, and dispensing the balls at timed intervals to a patient. These types of known devices, as in Brown, Carlson, and Stambuk, dispense substances arbitrarily, without taking into account the unique personal factors of each user and thus, fail to dispense a unique customized substance mixture for each user.

Furthermore, known diagnostic devices and methods are concerned with the act of diagnosing various illnesses and/or diseases present in a body, but are not designed to diagnose varying levels of pharmaceutical substances in the body with the use of a sample (e.g., blood, saliva, hair, or eye scan) in combination with a dispensing system. As such, pharmaceuticals administered to the user are not customized based on such a sample.

U.S. Pat. No. 6,063,026 to Schauss discloses a medical diagnostic analysis system comprising of a database containing a correlation of a plurality of diseases with a plurality of indicators associated with each such disease, a database including human experience test results associated with each indicator. An individual's test results are then compared with the second database data to determine presence levels for each indicator providing a method for correlating such factors with the various test indicators to identify therapeutic and/or contraindicated treatments and drugs. Also, U.S. Pat. No. 5,981,294 to Blatt, Mangan, Patel, and Allen discloses a device for blood separation in a diagnostic device comprising of a filter for separating red blood cells from a whole blood sample to form plasma. The filter includes a solid phase support and an agglutinin for red blood cells. The agglutinin is insoluble in the whole blood sample and is immobilized on the solid phase support. These types of known devices, as in Schauss and Blatt, et al, do not incorporate diagnostic samples in order to customize pharmaceutical mixtures for individual users, with potential use in a multi-user environment.

Additional deficiencies with known dispensing devices include the inability of such devices to automatically monitor the amounts of substances dispensed, order substance refills, connect to a network to obtain substance information, updates, and other pertinent substance information, and collect and store relevant user data.

Accordingly, there is a need in the art for a way to overcome the problems associated with pharmaceutical dispensing devices.

### Brief Description of the Drawings

The various features of the invention will best be appreciated by simultaneous reference to the description which follows and the accompanying drawings, in which:

FIG. 1 is a perspective view of the exterior of a dispenser in accordance with an embodiment of the present invention;

FIG. 2 is a perspective view of the interior of a dispenser in accordance with an embodiment of the present invention; and

FIG. 3 is a simplified flowchart of an embodiment of a method of the present invention for blending a customized pharmaceutical substance mixture for individual users.

### Detailed Description

Embodiments of the present invention include a method and an apparatus for dispensing controlled quantities of a customized pharmaceutical mixture for individual users based on various personal factors (e.g., age, gender, weight, daily level of physical activity, and current health status), with potential for being used in a multi-user environment. The customized pharmaceutical mixture may be comprised of nutritional supplements, such as vitamins, minerals, herbs, or medications, or any other pharmaceutical substance suitable for dispensing. The components of the mixture may be contained in individual compartments of a dispenser. The mixture may be dispensed in liquid, solid, powder, or gaseous form. Solid mixtures may vary in size and shape depending on the application. Examples include micro-capsule and pill sizes.

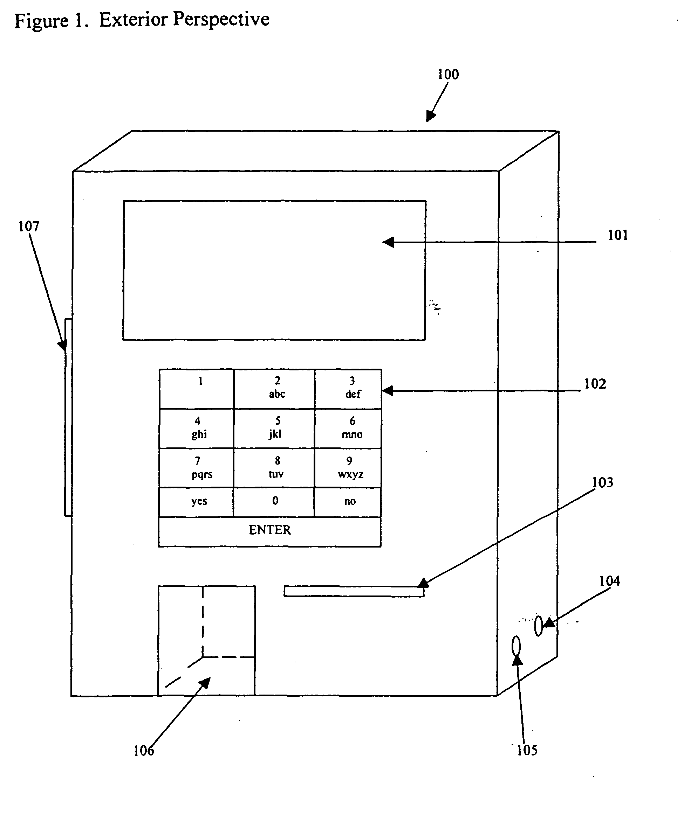

FIG. 1 illustrates an exterior view of an embodiment for a dispenser 100 in accordance with the principles of the present invention. As can be seen in FIG. 1, dispenser 100 may be comprised of a display screen 101 for viewing data stored in the dispenser 100 apparatus, a user interface 102 for inputting data into the apparatus, a drive interface 103 for transferring data between such apparatuses, a diagnostic device or outside network interface 104 for connecting a diagnostic device to dispenser 100 or connecting dispenser 100 to an outside network, a power supply interface 105 for providing power to dispenser 100, a collection area 106 for retrieving the personalized mixed substance, and a handle 107 for safe handling of dispenser 100 and for the removal of the front face of dispenser 100, providing access to the inside of dispenser 100.

The exterior of dispenser 100 may be colored with any color depending upon the desires of a particular purchaser.

The user interface 102 may be comprised of any type or style of keyboard devices, including, but not limited to, a touch phone keyboard system or a computer keyboard system, any type or style of voice interface, e.g., a microphone, or any other interface capable of providing a mechanism for the user to interact with the dispensing apparatus.

The drive interface 103 may accept any type of media, including, but not limited to, diskette, CD-ROM, or tape.

Another embodiment of dispenser 100 may include a component, such as heat-sealing bags, to store personalized substances for travel.

In an alternate embodiment, a diagnostic machine may be incorporated and connected to dispenser 100 via interface 104 to diagnose and quantify varying levels of pharmaceutical substance deficiencies or excess amounts in the body. The diagnosis may be accomplished through the analysis of an eye scan, breath, saliva, blood sample or any other such sample from the user. The diagnostic machine may use any diagnostic method to perform the analysis.

In another embodiment, a blood device may be connected to dispenser 100 via interface 104 to determine the presence of at least one of a plurality of analytes in a sample of whole blood.

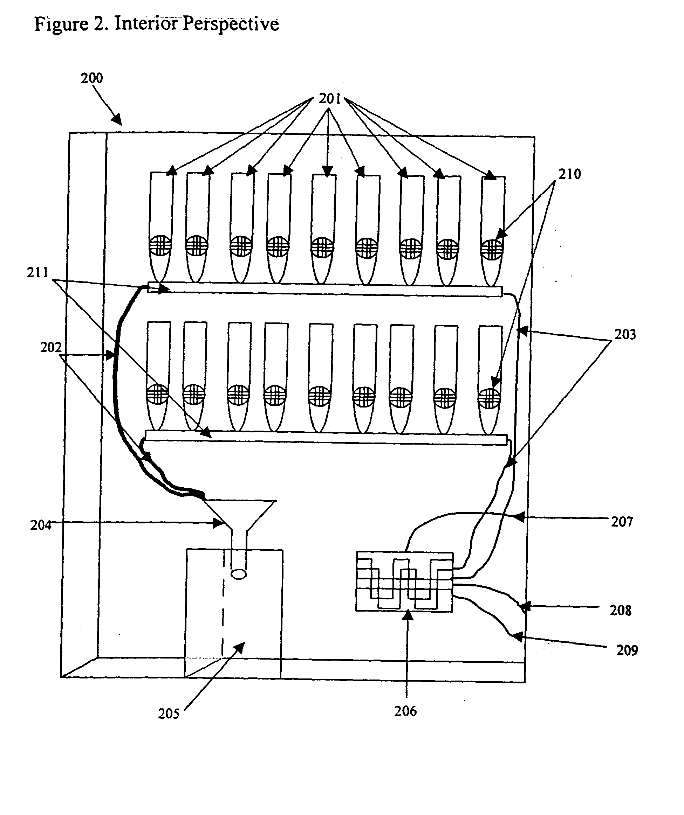

FIG. 2 illustrates an interior view of an embodiment for a dispenser 200 in accordance with the principles of the present invention. As can be seen in FIG. 2, dispenser 200 may be comprised of a plurality of containers 201, each containing a different pharmaceutical substance, each container having microchip 210, or any other type of keying mechanism, that stores data relevant for the substance enclosed in that container, each container being inserted into holding tray 211, passageway(s) 202 carrying those substances to the collection area 106 of FIG. 1, an accumulator 204 for mixing the dispensed substance into a personalized mixture, a collection area 205 for retrieving the personalized mixed substance, a logic unit (processor) 206 for controlling all electronic process, plurality of cables 203 for carrying data between the processor 206 and the containers 201, a cable 207 for carrying data between the processor 206 and the user interface 102 from FIG. 1, a cable 208 for carrying data between the processor 206 and a diagnostic device or outside network interface 104 from FIG. 1, and a cable 209 for carrying the power supply to the processor 206.

The plurality of containers 201, used to store pharmaceutical substances, may be arranged in any order inside dispenser 200 and may be of different sizes and shapes. In addition, the plurality of containers 201 interfaces with the dispenser 200 apparatus through the use of the keying mechanism. The apparatus may use electrical keying, where a microchip 210 may be attached to each container 201, and used to communicate between the said container and the apparatus after the said container is inserted into the holding tray 211, mechanical keying, where a mechanical design may only allow a given container 201 to be inserted into a given position in the holding tray 211, color coded keying, where containers 201 and their appropriate place in the holding tray 211 may be color coded with the same color, optical keying, where optical devices, e.g., bar codes, may be attached to the containers 201 and given positions in the holding tray 211 and the devices scanned and matched to indicate the given position of the containers 201 in the holding tray 211, or other keying method. "Keying" prevents the user from inserting a container 201 into the wrong position in the holding tray 211. In addition, "keying" enables the apparatus to continually monitor the location of any container 201 in the holding tray 211.

The substance dispensed from each container 201 may be measured by a precision measuring system to insure that the desired amount of said substance is dispensed from said container. By way of example only, a substance in the form of a powder may be measured and dispensed according to weight, a substance in the form of a liquid may be measured and dispensed according to volume, a substance in the form of a solid (e.g., micro-pellets) may be measured and dispensed according to count or weight.

The passageway(s) 202, used to carry substances to the collection area 106, may take on any embodiment, including, but not limited to, hollow cables or troughs.

The accumulator 204, used to create a unique mix of the dispensed substance, may be any form to direct the mix to the appropriate location, and may be the shape of a funnel, for example, or any other accumulating or mixing device.

The diagnostic device or outside network interface 104 may accept input from a diagnostic device or an outside network source and relay the input to the processor 206.

The handle 107 enables the removal of the front face of dispenser 200 providing easy access to containers 201 in order to replace depleted containers.

Individual components, in their pure state or mixed, may be contained in individual containers 201 in dispenser 200, with refills of said components available for purchase at a store or over a network. The replacement rate of individual containers 201 may depend on the rate of use of each component in the device in its respective container.

The various components (e.g., vitamins, multi-vitamins, minerals, botanicals, herbals, health foods, dietary supplements, nutritional supplements, and pharmaceuticals) may be added to the predetermined mixture using accurate measuring mechanisms. The mixture may be dispensed for consumption in the form of a pill, liquid, or dropped into a cup in an alternative form.

The mixture, comprising of the individual components stored in the apparatus, may be determined based on personal user factors, which also may be stored in the appropriate memory, that are input through the use of an interface such as a keyboard or a microphone. Each dispensed mixture may be based on the said unique factors and needs of individual users based on, in part, information input into the device by the user. An embodiment of the present invention may display labeling information for each component of a mixture.

Another embodiment of the present invention may provide an accurate measure of the exact nutritional supplements each user requires on a daily basis, under varying personal physical conditions. Accordingly, each user may be able to obtain a personalized mixture, thus, preventing the problem of consuming too much or too little of a given substance, reducing the amount and variety of nutritional supplements consumers buy, and reducing consumer costs. Instead of purchasing an array of generic vitamins targeted at a broad consumer group, such as children, males, females, seniors, and athletes, each household according to an embodiment of the present invention may invest in supplements that apply to each individual household member's needs. Alternatively, a single user household, which may include single or multiple individuals, may apply a personalized pharmaceutical mixture to that household to meet the needs of the household.

In addition, an embodiment of the present invention may allow a single or multiple users to replace numerous different nutritional supplement bottles with a single device, thus increasing shelf space and reducing the confusion of self-managing one's nutritional supplement needs and intake.

An alternate embodiment may remind users when it is time to obtain refills of various pharmaceutical substances that comprise a given mixture, and may enable automatic refill orders.

Another alternate embodiment may include flavoring components for the purpose of augmenting the flavor of the dispensed substance.

According to an embodiment of the present invention, a customized pharmaceutical mixture may be dispensed to a user with a liquid supply. A dispensing apparatus may introduce and mix the components of the mixture into a liquid supply. The components of the mixture may be determined based on various personal factors of a user. Accordingly, the mixture having the predetermined components consistent with personal factor considerations then may be dispensed in liquid form to the user. The user may then receive the pharmaceutical mixture at any time the user accesses the liquid supply.

Exemplary liquids that may comprise a mixture include water, orange juice, milk, and any other consumable liquid substance.

Alternatively, a customized pharmaceutical mixture may be dispensed to a user with a gas or solid supply. Similarly, the components of the mixture may be introduced and mixed into a gas or solid supply and then dispensed to the user. The user may then receive the mixture at any time the user accesses the supply.

Exemplary applications include dispensing pharmaceutical mixtures with a water supply to a household, school, or community.

Another embodiment of the present invention may store information and produce reports allowing the user to keep track of the pharmaceutical substances used in a given time period. An alternate embodiment may also remind the user when to take a given substance. Each person in a household may be able to store their unique information and specific requirements.

In another alternate embodiment of the present invention, processor 206 may include software that is updateable, for example, from a network or a disk. The updates may include information about new pharmaceutical substances or findings regarding such substances, their benefits, and recommended daily allowance levels. These updates may come from a pharmacy, a doctor, a pharmaceutical manufacturer, or any other source.

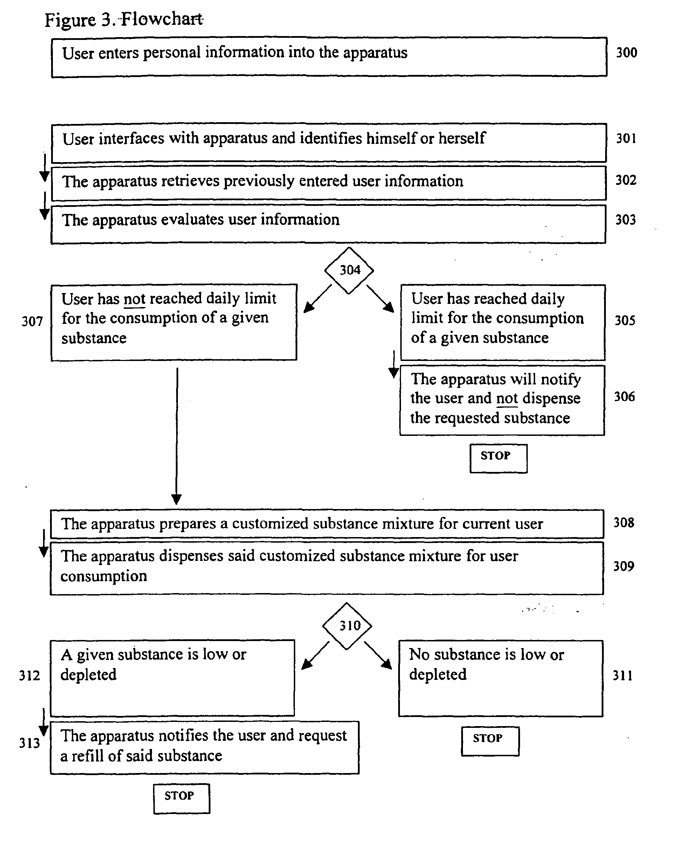

FIG. 3 illustrates a simplified flowchart of an embodiment of a method according to the present invention for blending a customized pharmaceutical substance mixture for individual users.

First, each user inputs personal information (e.g., age, gender, weight, daily level of physical activity, and current health status) into the apparatus (box 300), with the use of a keyboard, a microphone, or other interface. The interface may be active, such that the user interactively inputs the personal information, e.g., the user types on a keyboard or speaks into a microphone. Alternatively, the interface may be passive, such that the apparatus retrieves the personal information from the user as the user waits passively, e.g., the apparatus scans the user's retina or fingerprints. In another alternative, the interface may receive the personal information from a floppy disk or a network. Each time a given user wishes to obtain a substance mixture, said user must identify himself or herself to the apparatus (box 301) with the interface. The apparatus retrieves information on said user (box 302) from its memory bank and evaluates said user information (box 303) against statistical tables with recommended daily allowance values to determine the content and amount of substance to mix for said user. The apparatus inquires whether the user has reached the daily limit for consumption of a given substance (decision point 304). If said user has reached the daily limit for the consumption of a given substance (box 305), the apparatus notifies the user and does not dispense the substance (box 306). If said user has not reached the daily limit for the consumption of a given substance (box 307), the apparatus prepares a customized substance mixture for said user (box 308) based on stored user information and dispenses said customized substance mixture for said user consumption (box 309). The apparatus then inquires whether a given substance is low or depleted (decision point 310). If a given substance is low or depleted (box 312), the apparatus notifies the user and request a refill of said substance (box 313). If no substance is low or depleted (box 311), no action is taken.

The arrangement of the blocks of the flowchart shown in FIG. 3 represents one possible sequence for the activities described therein, but should not be construed as limited to that particular sequence. Other sequences for these activities are possible and considered to be within the scope of the invention.

The mechanisms and methods of embodiments of the present invention may be implemented using a general-purpose microprocessor programmed according to the teachings of the embodiments. The embodiments of the present invention thus also includes a machine readable medium, which may include instructions, which may be used to program a processor to perform a method according to the embodiments of the present invention. This medium may include, but is not limited to, any type of disk including floppy disk, optical disk, and CD-ROMs.

It is to be understood that the structure of the software used to implement the embodiments of the invention may take any desired form, such as a single or multiple programs. It is to be further understood that the method of an embodiment of the present invention may be implemented by software, hardware, or a combination thereof.

The disclosed embodiments are illustrative of the various ways in which the present invention may be practiced. Other embodiments can be implemented by those skilled in the art without departing from the spirit and scope of the present invention.

## Claims

1. An apparatus for dispensing a customized pharmaceutical mixture, comprising:
a plurality of containers containing multiple pharmaceutical substances; and
a controller in communication with the containers, the controller configured to select at least one of the containers, adjust amounts of the substances to be dispensed from the selected containers, and control the amounts dispensed to produce the customized pharmaceutical mixture,
wherein the amounts dispensed are determined based on particular health information of a user.

2. The apparatus of Claim 1, wherein the containers are identified by mechanical, electrical, magnetic, or optical indicators on the containers.

3. The apparatus of Claim 1 or 2, wherein the substances in the containers correlate to mechanical, electrical, magnetic, or optical indicators on the containers to ensure correct selection by the controller.

4. The apparatus of any of Claims 1 to 3, further comprising:
a support member,
wherein the containers are detachably connected to the support member and are replaceable.

5. The apparatus of any of Claims 1 to 4, further comprising:
a user interface configured to receive interactively the health information from the user of the pharmaceutical mixture.

6. The apparatus of any of Claims 1 to 5, wherein the controller is further configured to receive software updates regarding the pharmaceutical mixture.

7. The apparatus of any of Claims 1 to 6, wherein the controller is programmable.

8. The apparatus of any of Claims 1 to 7, wherein the controller is further configured to store health information of a plurality of users and to retrieve the stored health information of a particular user when the particular user wants the pharmaceutical mixture.

9. The apparatus of any of Claims 1 to 8, wherein the controller is further configured to remind the user to take or that the user has taken the pharmaceutical mixture.

10. The apparatus of any of Claims 1 to 9, wherein the mixture includes at least one of vitamins, multi-vitamins, minerals, botanicals, herbals, health foods, dietary supplements, nutritional supplements, and pharmaceuticals.

11. An apparatus for dispensing a customized pharmaceutical mixture, comprising:
a support member;
a plurality of containers detachably connected to the support member, the containers containing multiple pharmaceutical substances;
a user interface configured to receive input interactively from a user of the pharmaceutical mixture, the input including particular health information of the user; and
a processor in communication with the user interface and the containers, the processor configured to determine adjustments to amounts of the substances dispensed from the containers to produce the pharmaceutical mixture based on the input.

12. The apparatus of Claim 11, wherein the containers are replaceable.

13. The apparatus of Claim 11 or 12, wherein the processor is further configured to store health information of a plurality of users and to retrieve the stored health information of a particular user when the particular user wants the pharmaceutical mixture.

14. The apparatus of any of Claims 11 to 13, wherein the health information is input through passive identification of the user.

15. The apparatus of any of Claims 11 to 14,wherein the health information is input from a diagnostic device, the device determining the health information from a physical sample from the user.

16. The apparatus of any of Claims 11 to 15, wherein the mixture includes at least one of vitamins, multi-vitamins, minerals, botanicals, herbals, health foods, dietary supplements, nutritional supplements, and pharmaceuticals.

17. An apparatus for dispensing a customized pharmaceutical mixture, comprising:
a plurality of containers containing multiple pharmaceutical substances; and
a programmable controller in communication with the containers, the controller configured to receive program updates regarding the pharmaceutical substances and to adjust amounts of the substances dispensed to produce the pharmaceutical mixture based on the updates.

18. The apparatus of Claim 17, further comprising:
a user interface configured to receive interactively particular health information from a user of the pharmaceutical mixture,
wherein the health information is input through passive identification of the user or from a diagnostic device, the device determining the health information from a physical sample from the user, and
wherein the health information is used to adjust the amounts of the substances dispensed.

19. The apparatus of Claim 17 or 18, wherein the updates include information about new pharmaceutical substances and recommended daily allowance amounts,
wherein the updates are sent to the controller via a network or a disk and are sent from a pharmacy, a doctor, or a manufacturer.

20. An apparatus for dispensing a customized pharmaceutical mixture, comprising:
a support member;
a plurality of containers detachably connected to the support member, the containers containing multiple pharmaceutical substances, wherein each container is keyed into a particular location of the support member;
a user interface accessible by a plurality of users, the user interface configured to receive input interactively from one of the users of the pharmaceutical mixture, the input including particular health information of the user;
a processor in communication with the containers and the user interface, the processor configured to select at least one of the containers, determine adjustments to amounts of the substances to be dispensed from the selected containers, and control the amounts dispensed based on the input;
a mixer configured to mix the amounts of the substances from the selected containers to produce the pharmaceutical mixture; and
a collection area configured to receive the pharmaceutical mixture.

21. A method for dispensing a customized pharmaceutical mixture, comprising:
receiving particular health information interactively from a user of the pharmaceutical mixture;
determining amounts of components of the pharmaceutical mixture to be dispensed based on the health information; and
automatically mixing the components to produce the mixture.

22. The method of Claim 21, wherein the components include vitamins, multi-vitamins, minerals, botanicals, herbals, health foods, dietary supplements, nutritional supplements, and pharmaceuticals.

23. The method of Claim 21, wherein the components include combinations or alterations of vitamins, multi-vitamins, minerals, botanicals, herbals, health foods, dietary supplements, nutritional supplements, and pharmaceuticals.

24. The method of any of Claims 21 to 23, wherein the health information is input through passive identification of the user.

25. The method of any of Claims 21 to 23, wherein the health information is input from a diagnostic device, the device determining the health information from a physical sample from the user.

26. The method of any of Claims 21 to 25, further comprising:
reporting to the user an analysis of the health information; and
reporting to the user a diagnosis based on the health information.

27. The method of any of Claims 21 to 26, further comprising:
recognizing when a component is depleted;
notifying the user that the depleted component needs to be replenished; and
automatically ordering the depleted component via a network.

28. The method of any of Claims 21 to 27, further comprising:
storing health information of a plurality of users; and
retrieving the stored health information for one of the users when the one user wants the pharmaceutical mixture.

29. The method of any of Claims 21 to 28, further comprising:
reminding the user to consume or that the user has already consumed the pharmaceutical substance.

30. The method of any of Claims 21 to 29, wherein the mixture is dispensed in the form of a liquid, a solid, a powder, or a gas.

31. A method for dispensing a customized pharmaceutical mixture, comprising:
determining amounts of components of the pharmaceutical mixture to be dispensed based on particular health information of a user;
selecting at least one of a plurality of containers which contain the components; and
dispensing the determined amounts from the selected containers to produce the mixture.

32. The method of Claim 31, further comprising:
receiving the health information interactively from the user of the pharmaceutical mixture,
wherein the health information is input through passive identification of the user or from a diagnostic device, the device determining the health information from a physical sample from the user.

33. The method of Claim 31 or32, wherein the containers are identified by mechanical, electrical, magnetic, or optical indicators on the containers.

34. The method of any of Claims 31 to 33, wherein the components in the containers correlate to mechanical, electrical, magnetic, or optical indicators on the containers to ensure correct selection.

35. The method of any of Claims 31 to 34, further comprising:
notifying the user when a component in a container is depleted,
wherein the container is replaceable.

36. A method for dispensing a customized pharmaceutical mixture, comprising:
determining the nature of the pharmaceutical mixture based on particular health information of a user of the mixture;
determining components of the mixture based on the determined nature;
measuring amounts of the determined components;
dispensing the measured amounts from a plurality of containers, each container containing one of the determined components;
mixing the dispensed amounts together to form the mixture; and
dispensing the mixture to the user.

37. A machine readable medium containing program instructions for dispensing a customized pharmaceutical mixture which, when the instructions are executed by a processor, cause the processor to:
determine the nature of the pharmaceutical mixture based on particular health information of a user of the mixture;
determine components of the mixture based on the determined nature;
select containers, each container containing one of the determined components; and
control dispensing of the determined components to produce the mixture.

38. An apparatus, comprising:
means for separately containing different pharmaceutical substances;
means for inputting particular health information of a user; and
means for adjusting amounts of the substances dispensed from the containing means based on the health information.

39. A method for dispensing a customized pharmaceutical mixture, comprising:
selecting amounts of components of the pharmaceutical mixture to be dispensed;
mixing the components together into a liquid supply to produce the mixture; and
dispensing the liquid mixture to the user.

40. The method of Claim 39, wherein the user includes a single individual or a single household with multiple individuals.

41. The method of Claim 39 or 40, wherein the liquid supply includes water.

42. The method of any of Claims 39 to 41,further comprising:
receiving the health information interactively from the user of the pharmaceutical mixture,
wherein the health information is input through passive identification of the user.

43. The method of any of Claims 39 to 42, wherein the components include vitamins, multi-vitamins, minerals, botanicals, herbals, health foods, dietary supplements, nutritional supplements, and pharmaceuticals.

44. The method of any of Claims 39 to 43, further comprising:
mixing a flavoring component to the mixture to augment the flavor of the mixture.

45. The method of any of Claims 39 to 44, wherein the components of the mixture are dispensed in liquid, solid, powder, or gaseous form.
